# EUROPEAN PATENT SPECIFICATION

(11) **EP 1 785 142 B1**
(45) Date of publication and mention of the grant of the patent: **22.12.2010**
(21) Application number: 06012445.0
(22) Date of filing: 16.06.2006
(51) Int. Cl.: A61K 33/24, A61P 13/12, A61P 3/14, A61P 5/20

(54) **Treatment of chronic kidney disease (CKD) subjects using lanthanum compounds**
Behandlung von chronischen Nierenerkrankungen mit Lanthanum-Verbindungen
Traitement de nephropathies chroniques avec de composés à base de lanthanum

(30) Priority: 09.11.2005 US 272563
(43) Date of publication of application: 16.05.2007
(73) Proprietor: Shire International Licensing B.V., 1076 EE Amsterdam (NL)
(72) Inventor: Pratt, Raymond Dennis, Wayne PA 19087 (US); Webster, Isobel, Malborough Wiltshire SN8 4LB (GB); Damment, Stephen J.P., Landford Salisbury SP5 2ED (GB)
(74) Representative: Harrison Goddard Foote

(56) References cited:
- EP-A- 1 708 723
- US-A1- 2004 161 474
- US-A1- 2005 079 135
- CHIANG S S ET AL: "Lanthanum carbonate (Fosrenol) efficacy and tolerability in the treatment of hyperphosphatemic patients with end-stage renal disease." CLINICAL NEPHROLOGY. JUN 2005, vol. 63, no. 6, June 2005 (2005-06), pages 461-470, XP009074299 ISSN: 0301-0430
- CULLEL-YOUNG M ET AL: "LANTHANUM CARBONATE TREATMENT OF HYPERPHOSPHATEMIA" DRUGS OF THE FUTURE, BARCELONA, ES, vol. 28, no. 3, 1 March 2003 (2003-03-01), pages 224-228, XP009014864 ISSN: 0377-8282
- STIGANT CAROLINE ET AL: "Nephrology: 4. Strategies for the care of adults with chronic kidney disease." CMAJ : CANADIAN MEDICAL ASSOCIATION JOURNAL = JOURNAL DE L'ASSOCIATION MEDICALE CANADIENNE. 10 JUN 2003, vol. 168, no. 12, 10 June 2003 (2003-06-10), pages 1553-1560, XP002405225 ISSN: 0820-3946
- GABBIANI G ET AL: "Soft-tissue calcification induced by rare earth metals and its prevention by sodium pyrophosphate." BRITISH JOURNAL OF PHARMACOLOGY AND CHEMOTHERAPY. MAY 1966, vol. 27, no. 1, May 1966 (1966-05), pages 1-9, XP009074302 ISSN: 0366-0826
- HUTCHISON ALASTAIR ET AL: "Lanthanum carbonate versus calcium carbonate: Effect on parathyroid hormone (PTH) levels." JOURNAL OF THE AMERICAN SOCIETY OF NEPHROLOGY, vol. 14, no. Abstracts Issue, November 2003 (2003-11), page 205A, XP009074313 & MEETING OF THE AMERICAN SOCIETY OF NEPHROLOGY RENAL WEEK; SAN DIEGO, CA, USA; NOVEMBER 12-17, 2003 ISSN: 1046-6673

## Description

This invention relates to the treatment of subjects at risk for chronic kidney disease (CKD), having stage one to four CKD, susceptible to or suffering from soft tissue calcification associated with CKD, or susceptible to or suffering from secondary hyperparathyroidism, by orally administering a pharmaceutical composition containing a therapeutically effective amount of a non-toxic lanthanum compound.

### 2. BACKGROUND OF THE INVENTION

Chronic kidney disease (CKD) is a worldwide public health problem. According to a National Health and Nutrition Examination Survey (NHANES), the number of CKD subjects in the United States will increase from approximately 26 million in 2004 to approximately 40 million in 2020. One of the major complications of CKD is elevated blood phosphate levels resulting from the inability of the kidney to remove phosphate from the body by urine secretion. Excess phosphate levels in the blood result in CKD subjects developing hyperphosphatemia. The number of CKD subjects with hyperphosphatemia in the United States will increase from approximately 1 million in 2005 to approximately 2.8 million in 2020.

Currently, the Food and Drug Administration (FDA) has limited the treatment of hyperphosphatemia using phosphate binders to subjects with "End-Stage Renal Disease" (ESRD), *i.e.,* stage five of CKD. This sub-population of CKD subjects represents only 1 % of the total CKD subject population.

Hyperphosphatemia in ESRD subjects can be controlled using calcium-based phosphate binders, sevelamer (*i.e.,* a positively-charged polymer available, *e.g.,* as Renagel^{®} Tablets (sevelamer hydrochloride) from Genzyme in Cambridge, MA), and aluminum-based binders. Subjects who receive calcium-based binders often are unable to achieve desired phosphate levels without exceeding their recommended daily intake of calcium and are burdened with the amount of drug they must take. Additionally, calcium-based binders may cause hypercalcemia and exacerbate ectopic calcification as described, *infra.* Subjects who are prescribed sevelamer also have an unmanageably large pill burden due to the lack of potency of this drug. Aluminum-based binders, although highly potent and efficacious, are associated with central nervous system and bone toxicity when used over long periods.

Lanthanum carbonate in the form of a chewable tablet (available as Fosrenol^{®} from Shire Pharmaceuticals, Wayne, PA) has also been approved by the FDA to treat hyperphosphatemia in ESRD subjects. Unlike other problematic phosphate binders, lanthanum carbonate-based binders are potent with a manageable dosing regimen, do not cause hypercalcemia, and are non-toxic over long periods.

U.S. Patent No. 5,968,976 (assigned to Shire Pharmaceuticals) discloses a pharmaceutical composition comprising a lanthanum carbonate hydrate having the formula La₂(CO₃)₃•xH₂O, where x has a value between 3 to 6, to treat hyperphosphatemia in ESRD subjects. Processes for preparing this composition and a method to treat hyperphosphatemia in ESRD subjects using this composition are also described.

EP 1708723 discloses a method of treating hyperphosphataemia using lanthanum hydroxycarbonate. The same active ingredient is also stated to be useful in the treatment of stage 1 to stage 5 CKD.

However, there continues to be a need in the art to treat subjects who are at risk for CKD or who have varying forms of CKD not amounting to ESRD. This invention is based on the surprising finding that such subjects and subjects susceptible to or suffering from soft tissue calcification associated with CKD or secondary hyperparathyroidism can also benefit from the administration of lanthanum compound-based phosphate binders.

### 3. SUMMARY OF THE INVENTION

In accordance with the present invention, a non-toxic lanthanum carbonate or lanthanum carbonate hydrate for use in the treatment of a subject having stage one to stage four CKD, and susceptible to or suffering from soft tissue calcification associated with CKD, comprising orally administering a pharmaceutical composition containing as an active ingredient a therapeutically effective amount of a non-toxic lanthanum compound. As indicated hereinafter, the invention is applicable to the treatment of subjects exhibiting one or more functional or structural abnormalities indicating risk for, susceptibility to, or informing the diagnosis of any of stages one to four of CKD, soft tissue calcification associated with such CKD, or secondary hyperparathyroidism. When a lanthanum compound is administered to such a subject, it is possible to reduce if not arrest the progress of CKD, soft tissue calcification associated with CKD.

The above features and many other attendant advantages of the invention will be better understood by reference to the following detailed description.

### 4. DETAILED DESCRIPTION OF THE INVENTION

### 4.1. General Definitions

As used herein, the terms "treat, " "treating," or "treatment" mean the prevention, reduction, amelioration, partial or complete alleviation, or cure of CKD, soft tissue calcification, secondary hyperparathyroidism, or other as yet undiscovered conditions requiring control of phosphate absorption. For example, treatment of a subject at risk for or having one of stages one to four of CKD can mean the reduction of abnormally high serum phosphate levels; the prevention of soft tissue calcification; or the reduction of abnormally elevated parathyroid hormone (PTH) levels.

The term "symptom(s)" of those at risk for or having CKD, soft tissue calcification associated with CKD, or secondary hyperparathyroidism may be any functional or structural abnormality experienced by a subject and indicating kidney dysfunction, *e.g.,* those described in **Section 4.3,** *infra.* Among other abnormalities, as an example, one or more of the following symptoms may indicate risk for or the presence of CKD: a creatinine concentration of above about 1.6 mg/dL, a blood urea nitrogen (BUN) of above about 20 mg/dL, a blood phosphate level of above about 4.5 mg/dL, any detectable amount of blood in the urine, a urine protein concentration above about 100 mg/dL, a urine albumin concentration above about 100 mg/dL, an intact parathyroid hormone (PTH) concentration in the blood of above about 150 pg/mL, or a glomerular filtration rate (GFR) of below about 90 mL/min/1.73 m².

Further, as used herein, the term "subject" refers to a mammal (*e.g*., any veterinary medicine patient such as a domesticated animal, such as a dog or cat), or a human patient.

The terms "about" or "approximately" mean within an acceptable range for the particular parameter specified as determined by one of ordinary skill in the art, which will depend in part on how the value is measured or determined, *e.g.,* the limitations of the measurement system. For example, "about" can mean a range of up to 20% of a given value. Alternatively, particularly with respect to biological systems or processes, the term can mean within an order of magnitude, preferably within 5-fold, and more preferably within 2-fold, of a value.

### 4.2. Lanthanum Compounds

Lanthanum compounds useful in the method of the invention include lanthanum salts, hydrates, and solvates.

Lanthanum salts which may be utilized include lanthanum carbonate, lanthanum carbonate hydrates, lanthanum hydroxycarbonate, lanthanum chloride, lanthanum acetate, lanthanum lactate, other organic salts of lanthanum, lanthanum oxide, and lanthanum hydride. Lanthanum hydroxycarbonate is further described in U.S. Provisional Patent Application No. 60/591,105, filed July 27, 2004, entitled "Method of Treating Hyperphosphataemia using Lanthanum Hydroxycarbonate." published as WO 2006/015055. Lanthanum acetate used in a method of this invention has a possible additional benefit of increasing the pH buffering capacity in the body.

Useful lanthanum carbonates or lanthanum carbonate hydrates have the formula; La₂(CO₃)₃•xH₂O, wherein x has a value from 0 to 10. Preferably, x has a value from 3 to 8, desirably from 3 to 6. Most preferably, x may have an average value of about between 4 and 5. The hydration level of the lanthanum compound can be measured by methods well known in the art, such as thermo gravimetric analysis (TGA).

### 4.3. Chronic Kidney Disease (CKD)

The National Kidney Foundation-Kidney Disease Outcomes Quality Initiative ("NKF-K/DOQI" or "K/DOQI," as referred to herein) has defined chronic kidney disease (CKD) as either (1) having kidney damage as defined by structural or functional abnormalities of the kidney for 3 months or longer with or without a decreased glomerular filtration rate (GFR) or (2) having a GFR of less than 60 mL/min/1.73 m² for 3 months or longer with or without kidney damage. Structural or functional abnormalities are manifested by symptoms such as either pathologic abnormalities or markers of kidney damage, including abnormalities identified in imaging studies or the composition of blood or urine.

Examples of markers of kidney damage include a plasma creatinine concentration of above about 1.6 mg/dL and a blood urea nitrogen (BUN) concentration of above about 20 mg/dL. Typically, both of these markers are elevated in individuals with CKD. Additional markers of kidney damage can include hematuria (*i.e.,* any detectable amount of blood in the urine), proteinuria (*i.e.,* protein concentrations in urine above about 100mg/dL), albuminuria (*i.e.,* albumin concentrations in urine above about 100 mg/dL), an intact parathyroid hormone (PTH) concentration in the blood above about 150 pg/mL, or blood phosphate levels of above about 4.5 mg/dL. One specific marker of kidney disease is a GFR rate above normal (*i.e*., a GFR above about 90 mL/min/1.73 m²), however a below normal GFR also indicates CKD.

K/DOQI has published guidelines that define five different stages of CKD (Am J Kidney Dis. 2001, 37(suppl 1):S1-S238). The following table provides a description of each of the five stages of CKD and the GFR ranges for each of the stages.

**Five Stages of Chronic Kidney Disease (CKD)**

| **Stage** | **Description** | **GFR (mL/min/1.73m²)** |
|---|---|---|
| | At risk | 90-120 (with CKD symptoms) |
| 1 | Kidney damage with normal or elevated GFR | ≥ 90 |
| 2 | Kidney damage with mildly reduced GFR | 60-89 |
| 3 | Moderately reduced GFR | 30-59 |
| 4 | Severely reduced GFR | 15-29 |
| 5 | Kidney Failure (ESRD) | < 15 (or dialysis) |

Hyperphosphatemia in CKD subjects has several secondary effects. When a subject suffers from hyperphosphatemia, excess serum phosphate precipitates serum calcium causing widespread ectopic extraskeletal calcification. Unwanted calcium deposits can occur in cardiovascular tissue, resulting in an increased risk of cardiovascular complications that often lead to death. Additionally, increased serum phosphate decreases intestinal calcium absorption. These two mechanisms work concurrently to reduce serum calcium levels.

A reduction in serum calcium levels can contribute to an increase in the production of parathyroid hormone (PTH) and to the development of secondary hyperparathyroidism. Furthermore, recent studies show that high phosphate levels can stimulate PTH production directly and lead to secondary hyperparathyroidism. Continual stimulation of PTH secretion induces hyperplasia of the parathyroid gland and may lead to a parathyroidectomy becoming necessary.

It is believed that the method of the present invention involving the administration of lanthanum compound phosphate binders not only reduces plasma phosphate levels but ameliorates the effects of CKD in subjects susceptible to or having any of stages one to four CKD, including hyperphosphatemia, ectopic extraskeletal calcification, serum hypocalcemia, and secondary hyperparathyroidism. It should however, be understood that this invention is not limited to any particular biochemical or physiological mechanism.

### 4.4. Methods of Treating Chronic Kidney Disease (CKD)

One embodiment of this invention is a method of treating a subject having a symptom or symptoms of chronic kidney disease (CKD), comprising administering to the subject a pharmaceutical composition containing as an active ingredient a therapeutically effective amount of a non-toxic lanthanum compound. As indicated above, the subject treated may be at risk for CKD or have any of stages one to four CKD as defined above. Subjects at risk for CKD or who have any of stages one to four CKD who may be treated may have one or more of the following symptoms: a blood phosphate level of above about 4.5 mg/dL, a plasma creatinine concentration of above about 1.6 mg/dL, a BUN of above about 20 mg/dL, any detectable amount of blood in the urine, a urine protein concentration above about 100 mg/dL, a urine albumin concentration above about 100 mg/dL, an intact parathyroid hormone concentration in the blood above about 150 pg/mL, an abnormal GFR, or combination thereof.

The present method may be utilized to prevent the progression of renal pathology, *e.g*., by treating a subject displaying one or more symptoms of stage one CKD to prevent the development of CKD in the subject or by treating a subject having stage one CKD to prevent progression of the disease to stage two CKD, and so on.

### 4.5. Methods of Preventing Calcification

Another embodiment of the present invention is a method of treating calcification of soft tissue associated with CKD in a subject having a symptom or symptoms of CKD, by administering to the subject a pharmaceutical composition containing as an active ingredient a therapeutically effective amount of a non-toxic lanthanum compound.

Calcification can occur in any soft tissue. Soft tissue can include arterial tissue, cardiac muscle, heart valves, joints, skin and breast tissue.

### 4.6. Methods of Treating Secondary Hyperparathyroidism

Hyperparathyroidism is defined as a disease in a subject having an intact PTH level of about 150 pg/mL or greater. The symptoms of hyperparathyroidism include hypocalcaemia (*i.e.*, a blood calcium level below about 8.5 mg/dL), hyperphosphatemia (*i.e.*, a blood phosphate level of above about 4.5 mg/dL), and bone disorders (*e.g.,* bone fractures or bone pain).

### 4.7. Administration of a Therapeutically Effective Amount of the Lanthanum Compound

The lanthanum compound orally administered to subjects in accordance with this invention is suitably administered in dosage forms varying from 125 to 2000 mg as elemental lanthanum. A typical dosage for an adult can be, *e.g.,* 375 mg-6000 mg daily. More preferably, the dosage is 375-3750 mg/day. The dose can be divided and taken with each meal, for example 250, 500, 750, or 1000 mg tablet, *e.g*., three times per day. Serum plasma levels can be monitored weekly and dosages can be modified until an optimal serum phosphate level is reached. Administration may be conducted in an uninterrupted regimen; such a regimen may be a long term regimen, *e.g*., a permanent regimen, for treating chronic conditions.

The lanthanum compound may be orally administered in the form of tablets, capsules, chewable formulations, or the like. Due to their renal problems, subjects with CKD need to limit their liquid intake. Therefore, a formulation of a lanthanum compound that can be taken with no or limited amounts of liquid is desirable. For example, a lanthanum compound, in the form of, *e.g.,* beads, crushed tablets, powder, or sieved granules, may be sprinkled on food.

The lanthanum compound is administered in formulations such that plasma levels of lanthanum are low, *e.g.,* at least as low as those provided by a mean concentration curve where Cₘₐₓ, Tₘₐₓ and AUC are preferably less than 1.5 ng/ml, about 12 hours, and less than 50 ng•hr/ml, respectively, for a dose of 3 g per day (e.g., 1 g three times a day). Preferably, the Cₘₐₓ and AUC are less than 1.1 ng/ml and less than 32 ng•hr/ml, and desirably, Cₘₐₓ and AUC are less than 0.5 ng/ml and less than 20 ng•hr/ml, for such dosage. Tₘₐₓ values are essentially unaffected by dose and Cₘₐₓ and AUC values vary linearly with dosage for oral dosages up to about 1500 mg/day. Cₘₐₓ and AUC values plateau for dosages above about 1500 mg/day. All of these parameters have their common meanings.

The excipients used in the formulation administered by the present invention should be suitable for administration to renally impaired subjects. The excipients may include diluents, binders, and lubricants/glidants; other agents such as disintegrants, colors, and flavors/sweeteners can be added to the formulation.

Suitable diluents can be chosen from dextrates, corn syrup, oligosaccharide, isomaltooligosaccharide, glucose, lycasin, xylitol, lactitol, erythritol, mannitol, isomaltose, polydextrose, dextrin, starch, fructose, xylitol, maltodextrin, maltitol, isomalt, lactose, sorbitol, microcrystalline cellulose (such as Avicel), sucrose based diluent-binders (such as Nutab, Di-Pac or Sugartab), confectioner's sugar, calcium sulfate dihydrate, calcium lactate trihydrate, hydrolyzed starches (such as Emdex or Celutab), dextrose (such as Cerelose), inositol, hydrolyzed cereal solids (such as Maltrons or Mor-Rex), amylose or glycine.

Useful lubricant/glidants and blending/flow agents can be chosen from, for example, magnesium stearate, talc, polyethylene glycol, silica, colloidal anhydrous silica, hydrogenated vegetable oils, glyceryl behenate or glyceryl monostearate.

It can be advantageous to incorporate an antioxidant, for example, ascorbic acid, butylated hydroxyanisole or hydroquinone in the formulations to enhance their storage life.

Tablet formulations may be coated according to methods well known in the art. As indicated below, the formulations may, if desired, incorporate one or more further active ingredients.

It will be understood that the amounts of the respective ingredients incorporated in the formulations and the duration of the treatment according to the invention will vary depending on the requirements for treatment of individual subjects. The precise dosage regimen will be determined by the attending physician or veterinarian who will, *inter alia,* consider factors such as body weight, age and specific symptoms. The physician or veterinarian may titrate the dosage of lanthanum compound administered to a subject to determine the correct dosage for treatment. For example, a physician can measure a symptom of CKD (*e.g.,* blood phosphate level) in a patient, prescribe a particular lanthanum dosage to a patient for a week, and evaluate after the week if the dosage is appropriate by measuring the same symptom.

Typically, when a subject has soft tissue calcification, the subject is administered a relatively high dosage of lanthanum for a short period followed by administering a relatively low lanthanum dosage.

U.S. application Serial No. 10/926,330 entitled "Pharmaceutical Formulation Comprising Lanthanum Compounds" filed August 26, 2004 and published as U.S. publication No. 2005/0079135 on April 14, 2005. Any lanthanum compound, formulation or dosage disclosed in that application can be used to treat subjects with symptoms of CKD.

### 4.7.1. Administration of a Combination Treatment comprising Lanthanum and Vitamin D

Often, a subject suffering from the symptoms of CKD is also vitamin D deficient because, his or her kidneys can no longer metabolize vitamin D prohormones into the active metabolite of vitamin D; and increased phosphate levels found in CKD subjects are believed to suppress the production of the active metabolite of vitamin D. In another embodiment of the present invention, the lanthanum compound, in combination with vitamin D or an analog of vitamin D, is administered to a subject suffering from the symptoms of CKD to alleviate vitamin D deficiency. Levels of 25-hydroxy vitamin D₂ are low at values less than about 16 ng/mL and replacement treatment aims for levels of greater than or equal to about 16 ng/mL. Levels of 1, 25-dihydroxy vitamin D2 are low at values less than about 22 pg/mL and replacement treatment aims for levels of greater than about 22 pg/mL.

Examples of vitamin D sources which may be so administered concurrently with the lanthanum compound in this invention include 1,25 dihydroxy-vitamin D, the active metabolite of vitamin D (calcitriol, rocalcitrol). Examples of suitable vitamin D analogs include doxercalciferol (Hectorol^{®}, available from Bone Care International, Middleton, WI), and paricalcitol (Zemplar^{®}, available from Abbott Laboratories, Abbott Park, IL).

Vitamin D can be formulated and administered using routes as described, *supra.* Vitamin D can be combined in the same formulation as the lanthanum compound or can be given in a different formulation as the lanthanum compound. As described above for the lanthanum compound, the precise dosage regimen for vitamin D will be determined by the attending physician or veterinarian who will, *inter alia,* consider factors such as body weight, age and specific symptoms. The physician or veterinarian may titrate the dosage of vitamin D administered to a subject to determine the correct dosage for treatment.

In a specific embodiment, 100 USP units of vitamin D is administered once per day and a lanthanum compound is administered three times per day to a subject requiring treatment.

### 4.7.2. Administration of a Combination Treatment comprising a Lanthanum Compound and a Calcium Source

As described above, CKD subjects often suffer from hypocalcaemia (*i.e*., a blood calcium concentration below about 8.5 mg/dL). In a further embodiment of this invention, a lanthanum compound is administered in combination with a calcium source to a subject suffering from the symptoms of CKD.

Examples of forms of calcium that can be co-administered with lanthanum include calcium carbonate (*e.g.,* Tums^{®} available from GlaxoSmithKline, Uxbridge, UK), calcium acetate (*e.g.,* PhosLo^{®} available from Nabi Biopharmaceuticals, Boca Raton, FL), and CaCl₂.

Calcium dosages (expressed as elemental calcium) can range from 1 to 1.5 grams/day. A calcium compound can be combined in the same formulation with the lanthanum compound or can be given in a different formulation as the lanthanum compound. A calcium compound, whether in the presence or absence of the lanthanum compound in the same formulation, can be formulated and administered using routes as described, *supra.* The exact dosage regimen for calcium will be determined by the attending physician or veterinarian who will, *inter alia,* consider factors such as body weight, age and specific symptoms. The physician or veterinarian may titrate the dosage of calcium administered to a subject to determine the correct dosage for treatment.

In a specific embodiment, 1-2 tablets containing calcium and the lanthanum compound are each given 3 times per day.

### 4.7.3. Administration of a Combination Treatment comprising Lanthanum and Vitamin K

A subject suffering from the symptoms of CKD can be vitamin K deficient. In another embodiment of the present invention, the lanthanum compound, in combination with vitamin K, is administered to a subject suffering from the symptoms of CKD to alleviate vitamin K deficiency.

Examples of vitamin K sources include vitamin K1 (phylloquinone), vitamin K2 (menaquinone), and vitamin K3 (menadione).

Vitamin K can be formulated and administered using routes as described, *supra.* Vitamin K can be combined in the same formulation as the lanthanum compound or can be given in a different formulation as the lanthanum compound. As described above for the lanthanum compound, the precise dosage regimen for vitamin K will be determined by the attending physician or veterinarian who will, *inter alia,* consider factors such as body weight, age, and specific symptoms. The physician or veterinarian may titrate the dosage of vitamin K administration to a subject to determine the correct dosage for treatment.

In a specific embodiment, 2.5 to 25 mg of vitamin K1 are administered once per day and a lanthanum compound is administered three times per day to a subject requiring treatment.

### 5. EXAMPLE: Reduction in Renal Calcification with Lanthanum Treatment

Crl:CD (SD) Br (VAFplus) female rats (available from Charles River Laboratories in Wilmington, MA) were orally dosed once daily with 0, 100, 500, or 1500 mg (salt)/kg of lanthanum carbonate for 104 weeks. Crl:CD (SD) Br (VAFplus) female rats develop spontaneous renal pathology, including mineralization, as they age. At week 105, the pathological examination of the kidneys of these rats was performed.

Approximately 50% (n=120) of the rats that were not dosed with lanthanum carbonate showed evidence of pelvic/papillary mineralization, whereas approximately only 35% (n=60), 5% (n=60), and 1.7% (n=60) of rats dosed with 100, 500 and 1500 mg (salt)/kg/day, respectively, demonstrated .evidence of pelvic/papillary mineralization. Furthermore, approximately 63% (n=120) of the rats that were not dosed with lanthanum carbonate showed evidence of transitional cell hyperplasia in their kidneys, whereas approximately only 48% (n=60), 23% (n=60), and 7% (n=60) of rats dosed with 100, 500 and 1500 mg (salt)/kg/day, respectively, demonstrated evidence of transitional cell hyperplasia. Transitional cell hyperplasia is a response to trauma caused by the unnatural presence of mineral in the tissue, thus mineralization and hyperplasia are closely linked.

This study indicates that the oral administration of lanthanum carbonate reduces renal calcification.

U.S. Application Serial No. 11/272,569 entitled "Stabilized Lanthanum Carbonate Compositions" filed November 9, 2005 is published as US 2006/0121127.

The present invention is not to be limited in scope by the specific embodiments described herein. Indeed, various modifications of the invention in addition to those described herein will be apparent to those skilled in the art from the foregoing description. Such modifications are intended to fall within the scope of the appended claims.

Also disclosed are the following special embodiments.
1. A method of treating a subject (1) at risk for chronic kidney disease (CKD), (2) having stage one to stage four CKD, (3) susceptible to or suffering from soft tissue calcification associated with CKD, or (4) susceptible to or suffering from secondary hyperparathyroidism, comprising orally administering to the subject a pharmaceutical composition containing as an active ingredient a therapeutically effective amount of a non-toxic lanthanum compound.
2. The method of embodiment 1, wherein the subject is a mammal.
3. The method of embodiment 2, wherein the subject is a human.
4. The method of embodiment 1, wherein the lanthanum compound is a lanthanum salt selected from lanthanum carbonate, lanthanum carbonate hydrate, lanthanum hydroxycarbonate, or lanthanum chloride.
5. The method of embodiment 4, wherein the lanthanum compound is a lanthanum carbonate or lanthanum carbonate hydrate having the formula:

   La₂(CO₃)₃•xH₂O

   wherein x has a value from 0 to 10.
6. The method of embodiment 1, wherein the effective amount of elemental lanthanum in the lanthanum compound is from about 375 mg/kg/day to about 3750 mg/kg/day.
7. The method of embodiment 1, wherein the subject has at least one of the following: a blood phosphate level of above about 4.5 mg/dL, a plasma creatinine concentration of above about 1.6 mg/dL, a blood urea nitrogen (BUN) of above about 20 mg/dL, any detectable amount of blood in the urine, a urine protein concentration above about 100 mg/dL, a urine albumin concentration above about 100 mg/dL, an intact parathyroid hormone (PTH) concentration in the blood of above about 150 pg/mL, or a glomerular filtration rate (GFR) of below about 90 mL/min/1.73 m².
8. A method of treating a subject at risk for chronic kidney disease (CKD) or having stage one to stage four CKD, comprising orally administering to the subject a pharmaceutical composition containing as an active ingredient a therapeutically effective amount of a non-toxic lanthanum compound.
9. A method of embodiment 8, wherein the subject is treated to reduce or arrest the progress of CKD.
10. A method of treating a subject susceptible to or suffering from soft tissue calcification associated with chronic kidney disease (CKD), comprising orally administering to the subject a pharmaceutical composition containing as an active ingredient a therapeutically effective amount of a non-toxic lanthanum compound.
11. The method of embodiment 10, wherein the soft tissue is arterial tissue, cardiac muscle, heart valves, skin, or a combination thereof.
12. A method of treating a subject susceptible to or suffering from secondary hyperparathyroidism, comprising orally administering to the subject a pharmaceutical composition containing as an active ingredient a therapeutically effective amount of a non-toxic lanthanum compound.
13. The method of embodiment 12, wherein the subject has an intact parathyroid hormone (PTH) concentration in the blood of above about 150 pg/mL.

## Claims

1. Non-toxic lanthanum carbonate or lanthanum carbonate hydrate in the form of an oral medicament for use in the treatment of a subject susceptible to or suffering from soft tissue calcification associated with CKD and who is at risk for or has stage one to stage four chronic kidney disease (CKD).

2. Non-toxic lanthanum carbonate or lanthanum carbonate hydrate as claimed in claim 1, wherein the subject is a mammal.

3. Non-toxic lanthanum carbonate or lanthanum carbonate hydrate as claimed in claim 2, wherein the subject is a human.

4. Non-toxic lanthanum carbonate or lanthanum carbonate hydrate as claimed in claim 1, wherein the lanthanum carbonate or lanthanum carbonate hydrate has the formula:
La₂(C0_{3˙}xH₂0
wherein x has a value from 0 to 10.

5. Non-toxic lanthanum carbonate or lanthanum carbonate hydrate as claimed in any one of claims 1-4, wherein the effective amount of elemental lanthanum in the lanthanum carbonate or lanthanum carbonate hydrate is from about 375 mg/day to about 3750 mg/day.

6. Non-toxic lanthanum carbonate or lanthanum carbonate hydrate as claimed in any one of claims 1-5, wherein the subject has at least one of the following: a blood phosphate level of above about 4.5 mg/dL, a plasma creatinine concentration of above about 1.6 mg/dL, a blood urea nitrogen (BUN) of above about 20 mg/dL, any detectable amount of blood in the urine, a urine protein concentration above about 100 mg/dL, a urine albumin concentration above about 100 mg/dL, an intact parathyroid hormone (PTH) concentration in the blood of above about 150 pg/mL, or a glomerular filtration rate (GFR) of below about 90 mL/min/1.73 m².

7. Non-toxic lanthanum carbonate or lanthanum carbonate hydrate as claimed in claim 1, wherein the soft tissue is arterial tissue, cardiac muscle, heart valves, skin, or a combination thereof.

## Patentansprüche

1. Nicht-toxisches Lanthancarbonat oder Lanthancarbonathydrat in Form eines oralen Arzneimittels zur Verwendung in der Behandlung eines Subjektes, das anfällig ist für eine Weichgewebekalzifizierung oder bereits hiervon betroffen ist, die mit CKD assoziiert ist, und für das das Risiko für ein chronisches Nierenversagen (CKD) des Stadiums 1 bis Stadium 4 besteht oder das bereits hiervon betroffen ist.

2. Nicht-toxisches Lanthancarbonat oder Lanthancarbonathydrat wie in Anspruch 1 beansprucht, wobei das Subjekt ein Säugetier ist.

3. Nicht-toxisches Lanthancarbonat oder Lanthancarbonathydrat wie in Anspruch 2 beansprucht, wobei das Subjekt ein Mensch ist.

4. Nicht-toxisches Lanthancarbonat oder Lanthancarbonathydrat wie in Anspruch 1 beansprucht, wobei das Lanthancarbonat oder Lanthancarbonathydrat folgende Formel aufweist:
La₂(CO₃)₃·xH₂O
wobei x einen Wert von 0 bis 10 aufweist.

5. Nicht-toxisches Lanthancarbonat oder Lanthancarbonathydrat wie in einem der Ansprüche 1 bis 4 beansprucht, wobei die wirksame Menge des elementaren Lanthans in dem Lanthancarbonat oder Lanthancarbonathydrat bei etwa 375 mg/Tag bis etwa 3750 mg/Tag liegt.

6. Nicht-toxisches Lanthancarbonat oder Lanthancarbonathydrat wie in einem der Ansprüche 1 bis 5 beansprucht, wobei für das Subjekt zumindest eines des Folgenden gilt: ein Blutphosphatspiegel von größer etwa 4,5 mg/dl, eine Plasmakreatinkonzentration von größer etwa 1,6 mg/dl, einen Blut-Harnstoff-Stickstoff (BUN) von größer etwa 20 mg/dl, eine beliebige nachweisbare Menge von Blut im Urin, eine Urinproteinkonzentration von größer etwa 100 mg/dl, eine Urinalbuminkonzentration von größer etwa 100 mg/dl, eine intakte Konzentration von Parathormon (PTH) im Blut von größer etwa 150 pg/ml, eine glomeruläre Filtrationsrate (GFR) von kleiner etwa 90 ml/min/1,73 m².

7. Nicht-toxisches Lanthancarbonat oder Lanthancarbonathydrat wie in Anspruch 1 beansprucht, wobei es sich bei dem Weichgewebe um arterielles Gewebe, Herzmuskel, Herzklappen, Haut oder eine Kombination hieraus handelt.

## Revendications

1. Carbonate de lanthane ou hydrate carbonate de lanthane non toxique, sous la forme d'un médicament oral pour utilisation en vue du traitement d'un sujet susceptible de souffrir ou souffrant de calcification de tissus souples associée à CKD et qui présente un risque d'être, ou est, entre les stades un et quatre de maladie de rein chronique (CKD).

2. Carbonate de lanthane ou hydrate carbonate de lanthane non toxique selon la revendication 1, **caractérisé en ce que** le sujet est un mammifère.

3. Carbonate de lanthane ou hydrate carbonate de lanthane non toxique selon la revendication 2, **caractérisé en ce que** le sujet est un être humain.

4. Carbonate de lanthane ou hydrate carbonate de lanthane non toxique selon la revendication 1, **caractérisé en ce que** le carbonate de lanthane ou hydrate carbonate de lanthane non toxique présente la formule :
La₂(CO₃)₃*xH₂O
Où x a une valeur de 0 à 10.

5. Carbonate de lanthane ou hydrate carbonate de lanthane non toxique selon l'une des revendications 1 à 4, **caractérisé en ce que** la quantité effective de lanthane élémentaire dans le carbonate de lanthane ou hydrate carbonate de lanthane non toxique est entre environ 375 mg/kg/jour jusqu'à environ 3750 mg/kg/jour.

6. Carbonate de lanthane ou hydrate carbonate de lanthane non toxique selon l'une des revendications 1 à 5, **caractérisé en ce que** le sujet présente au moins l'un des éléments suivants :
- un niveau de phosphate dans le sang au dessus d'environ 4,5 mg/dL
- une concentration de créatinine plasma au dessus d'environ 1,6 mg/dL
- un taux d'azote d'urée dans le sang (BUN) au dessus d'environ 20 mg/dL
- toute quantité détectable de sang dans l'urine
- une concentration de protéine dans l'urine au dessus d'environ 100mg/dL
- une concentration d'albumine dans l'urine au dessus d'environ 100 mg/dL
- une concentration d'hormone para thyroïde intacte (PTH) dans le sang, au dessus d'environ 150 pg/mL ; ou
- un taux de filtration glomérulaire (GFR) au dessous d'environ 90 mL/minutes/1,73m².

7. Carbonate de lanthane ou hydrate carbonate de lanthane non toxique selon la revendication 1, **caractérisé en ce que** le tissu souple est un tissu artériel, un muscle cardiaque, des valves de coeur, la peau, ou une combinaison de ces derniers.
